## Europäisches Patentamt

## European Patent Office

## Office européen des brevets

(11) Publication number: **0 042 246**
**B1**

## EUROPEAN PATENT SPECIFICATION

(45) Date of publication of patent specification: **18.03.87**

(21) Application number: **81302548.3**

(22) Date of filing: **09.06.81**

(51) Int. Cl.⁴: **C 07 K 13/00, A 61 K 45/02, C 12 P 21/02, C 12 N 15/00**

(54) **Plasmid.**

(30) Priority: **12.06.80 US 158721**

(43) Date of publication of application:
**23.12.81 Bulletin 81/51**

(45) Publication of the grant of the patent:
**18.03.87 Bulletin 87/12**

(84) Designated Contracting States:
**AT BE CH DE FR GB IT LI NL SE**

(56) References cited:
**EP-A-0 032 134**
**EP-A-0 034 306**
**EP-A-0 034 307**
**EP-A-0 041 313**
**EP-A-0 043 980**
**EP-A-0 048 970**
**GB-A-2 063 882**
**GB-A-2 068 970**

**VIROLOGY, vol.97, 1979, Academic Press W.E. STEWART et al.: "Effect of glycosylation inhibitors on the production and properties of human leukocyte interferon", pages 473-476**

(73) Proprietor: **CANCER INSTITUTE OF JAPANESE FOUNDATION FOR CANCER RESEARCH**
**37-1, Kamiikebukuro 1-chome**
**Toshimaku Tokyo (JP)**

(73) Proprietor: **PRESIDENT AND FELLOWS OF HARVARD COLLEGE**
**17 Quincy Street**
**Cambridge Massachusetts 02138 (US)**

(72) Inventor: **Guarente, Leonard P.**
**132 Oxford Street Apt. 3A**
**Cambridge, MA 02140 (US)**
Inventor: **Ptashne, Mark**
**89 Irving Street**
**Cambridge, MA 02138 (US)**
Inventor: **Roberts, Thomas M.**
**Five Irving Terrace**
**Cambridge, MA 02138 (US)**
Inventor: **Taniguchi, Tadatsugu**
**4-27-12 Tagara Nerima-Ku**
**Tokyo (JP)**

(74) Representative: **Moon, Donald Keith et al**
**BREWER & SON 5-9 Quality Court Chancery Lane**
**London WC2A 1HT (GB)**

Courier Press, Leamington Spa, England.

(59) References cited:

**THE JOURNAL OF BIOLOGICAL CHEMISTRY, vol.252, no.12, June 25, 1977, (US) E.A. HAVELL et al.: "Altered molecular species of human interferon produced in the presence of inhibitors of glycosylation", pages 4425-4427**

**PROC. NATL. ACAD. SCI., vol.77, no.9, September 1980, (US) T. TANIGUCHI et al.: "Expression of the human fibroblast interferon gene in escherichia coli", pages 5230-5233**

**NATURE, vol.287, no.5781, October 1980, Macmillan Journals Ltd., Chesham, Bucks (GB) D.V. GOEDDEL et al.: "Human leukocyte interferon produced by E. coli is biologically active", pages 411-416**

**Description**

This invention relates to the use of a plasmid in the production of antiviral agents, specifically, interferon, and to genes coding for such agents.

Human interferon is known to be a powerful antiviral agent, and undoubtedly has other therapeutic uses as well. The only form of inteferon presently available, the naturally-occurring glycosylated interferon prepared from human cell culture extracts, is very scarce and expensive, and its usefulness has consequently been extremely limited.

We have discovered that forms of human interferon can be made, and that, despite the fact that, unlike naturally-occurring interferon (IF), they are not glycosylated but rather are in the form of aglycons, they nonetheless can exhibit antiviral activity and can be used therapeutically. We found that extracts from *E. coli* containing one of the unglycosylated interferons of the invention, unglycosylated human fibroblast inteferon, effectively inhibited the cytopathic effect on cultured human fibroblast cells (FS7) normally produced by vesicular stomatitis virus.

Unglycosylated human inteferons are disclosed in EP—A—0034306 and 0041313, and in The Journal of Biological Chemistry, Vol. 252, No. 12, June 25, 1977 (US). The last reference is to an altered molecular species of human inteferon produced in the presence of inhibitors of glycosylation by human FS—4 fibroblast cells.

According to a first aspect of the present invention, there is provided plasmid pLG104R wherein 3700 base pair PstI—PstI region from TpIF319 bearing the 3' end of the interferon is ligated to the ~1050 base pair PstI—PstI region of pLG104 bearing the *lac* promoter adjacent to the ATG at the beginning of pre-interferon.

According to a second aspect of the present invention, there is provided plasmid pLG117R wherein 3700 base pair PstI—PastI region from TpIF319 bearing the 3' end of the interferon is ligated to the ~990 base pair PstI—PstI region of pLG117 bearing the *lac* promoter adjacent to the ATG at the beginning of mature interferon.

The plasmids of the present invention are constructed by inserting a gene coding for human fibroblast interferon into a vector plasmid. Such plasmids can be used for producing human fibroblast inteferon, with advantage over the prior use of fibroplast cells.

The unglycosylated IFs or aglycons produced by the use of the plasmids of the present invention can be administered to patients alone or dispensed or dissolved in any pharmaceutically-acceptable non-toxic carrier suitable for the desired mode of administration, which may be oral or parenteral, that is, by injection which is intravenous, intramuscular, intraperitoneal, or other conventional mode. The amount of aglycon which is effective varies over a wide range, depending upon the mode of administration and the result desired, and can readily be determined in any given case by simple screening procedures.

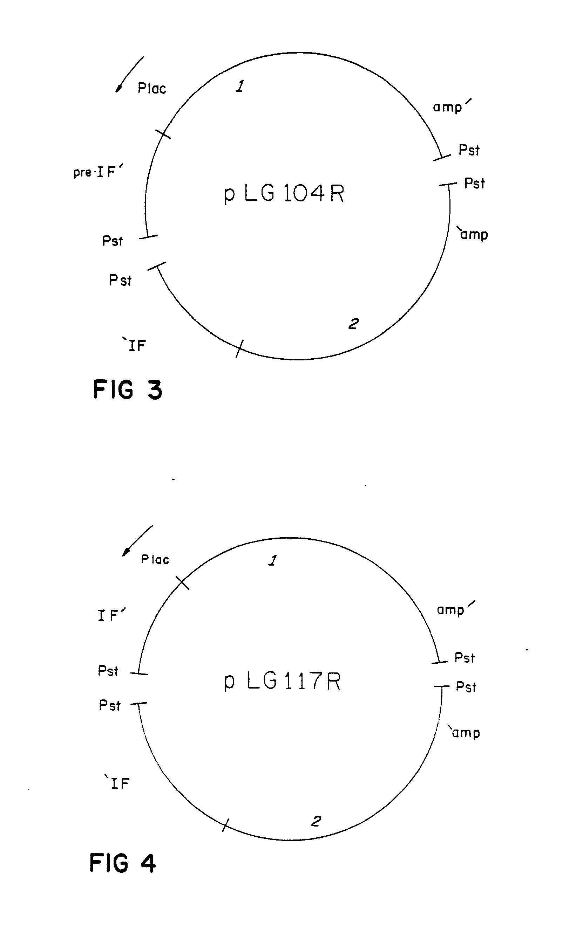

In the drawings, Figs. 1 to 4 show diagrammatic representations of fused genes used in various steps of one method of making one of the interferons of the invention, unglycosylated human fibroblast interferon. The fused genes of Figs. 3 and 4, capable of coding for this interferon, constitute part of the invention as well.

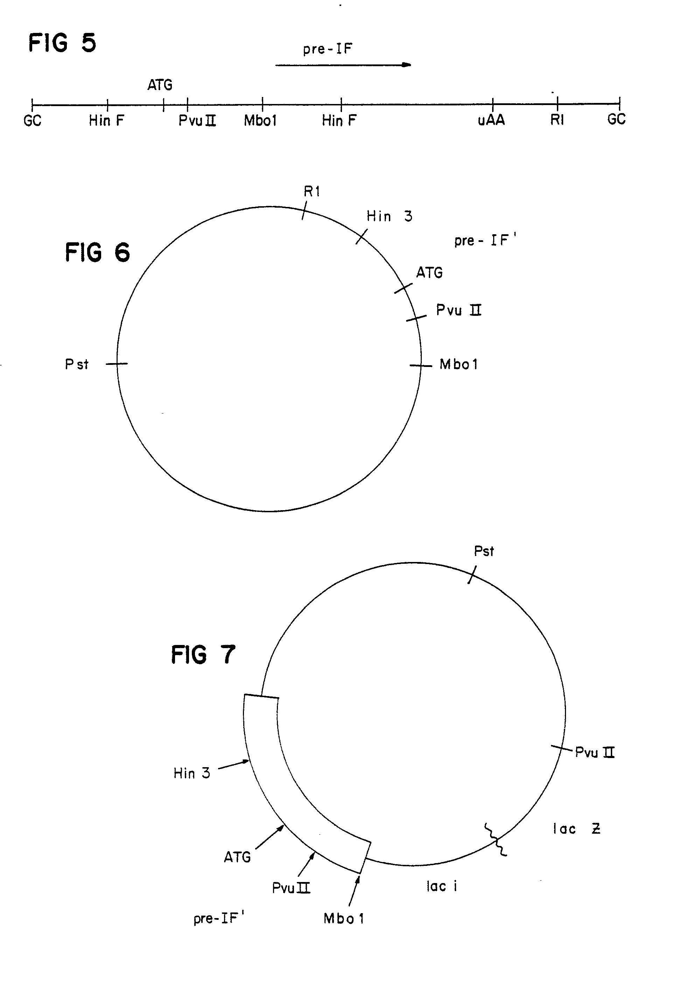

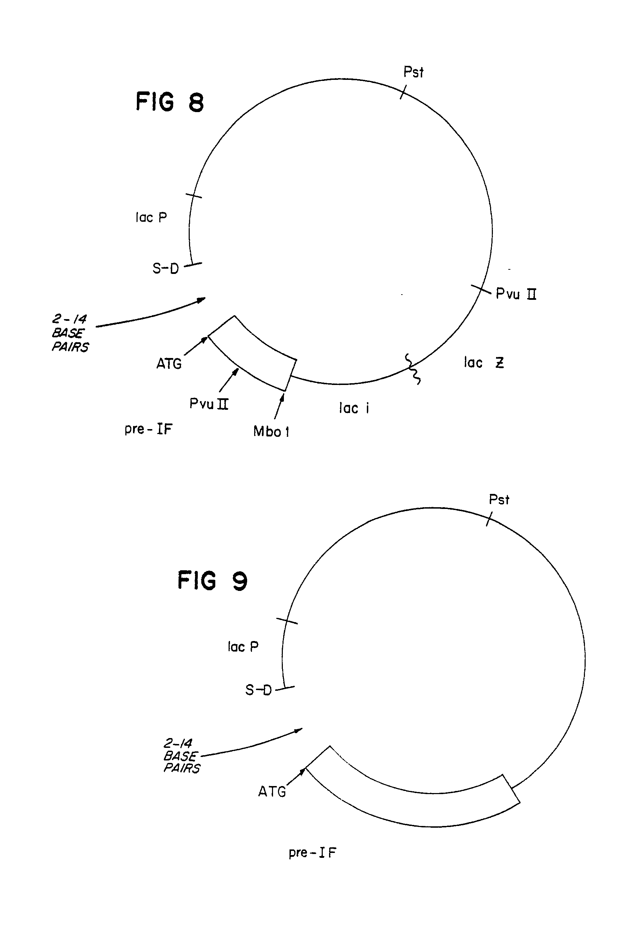

Figs. 5 to 9 show diagrammatic representations of fused genes used to make another inteferon of the invention, unglycosylated human leucocyte interferon. The fused gene of Fig. 9, capable of coding for this interferon, is also part of the present invention.

The following specific examples are intended to illustrate more fully the nature of the present invention without acting as a limitation upon its scope. Both describe unglycosylated inteferons that can be made according to a method described in our copending application entitled "Optical Polypeptide Production", filed March 17, 1980 and having Serial No. 131,152. That application hereby incorporated by reference, describes a method of providing a fused gene having a promoter located an optimal distance from the translation start site. The method involves, in one aspect, providing a fused gene having a region of a gene coding for a desired polypeptide fused to a region of a gene coding for an assayable polypeptide, inserting a portable promoter at varying distances in front of the translation start site, transforming microorganisms with the fused genes, selecting those producing the greatest amount of assayable polypeptide, and reconstituting the gene for the desired polypeptide.

Examples 1 and 2

We have used two variations of the above-described method to express, in *E. coli* strain K—12, the human fibroblast interferon gene isolated and described in Taniguchi et al. (1979) Proc. Jap. Acad. Sci, *55*, 464—469 and Taniguchi et al. (1980) Gene *10*, 11—15. Both variations employed β-galactosidase as the assayable polypeptide. According to one invention, synthesis of unglycosylated IF was initiated at the translation start site (ATG) at the beginning of the mature IF molecule. According to the second variation, synthesis was initiated at the ATG at the beginning of the leader sequence, twenty-one amino acids long, of pre-interferon. This second variation produced the same mature, active, unglycosylated IF as the first because, apparently *E. coli* cleaved off the leader sequence following synthesis.

Several of the steps of the two variations were identical, and the two will therefore be described together, and the differences pointed out where necessary.

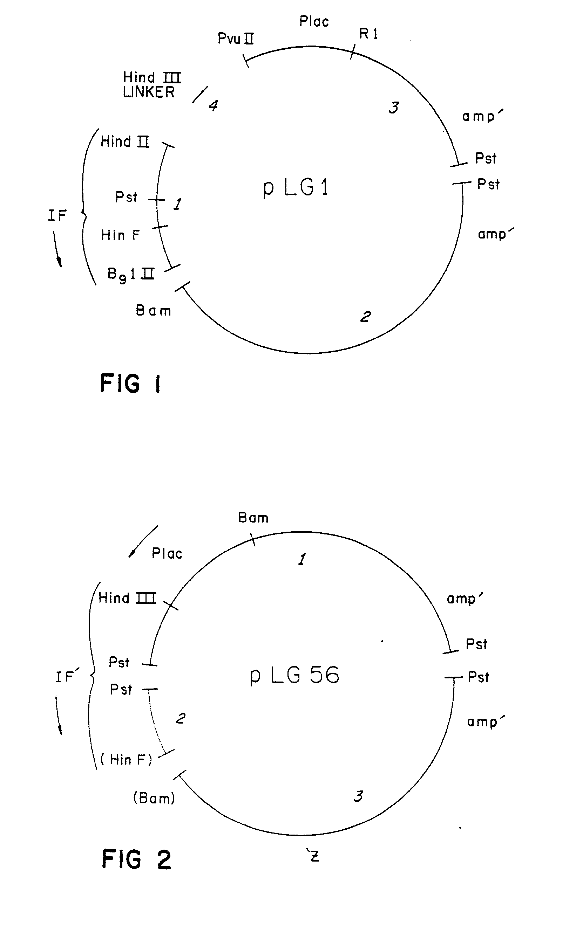

The first step was to construct the new plasmid, pLG 1, shown in Fig. 1, by ligating together the following four DNA fragments:

1. The ~550 base pair Hind II—BglII fragment from TpIF 319—13 (Taniguchi, *supra*).

2. The ~5,000 base pair Bam-Pst fragment from pLG 300 (Guarente et al. Serial No. 131,152, *supra*).

3. The ~850 base pair Pst—PvuII portable promoter fragment from pGL101; this fragment has a transcription start site and a Shine-Dalgarno sequence, AGGA.

4. The 10 base pair Hind III linker fragment.

*E. coli* were transformed with the ligation mix and selected for growth on ampicillin. A plasmid with the structure of pLG 1 was isolated from a transformed clone. Next, a 10 base pair Bam linker was inserted at the R1 of pLG 1 site by cutting at R1, filling in the cohesive ends with deoxynucleotides using DNA polymerase (Backman et al. (1976) Proc. Nat. Acad. Sci., USA 73, 4174—4178) an religating in the presence of Bam linkers. This yielded plasmid pLG 45. *E. coli* were again transformed and selected for growth on ampicillin, and plasmid pLG 56, shown in Fig. 2, was then constructed by ligating together the following DNA fragments:

1. The ~1050 base pair Pst—Pst fragment from the selected pLG 45 plasmids bearing the *lac* promoter and the 5' end of the IF gene.

2. The 75 base pair Pst—HinF fragment containing an interstitial portion of the IF gene from TpIF 319—13 and having a filled in HinF end.

3. The ~5Kb Bam—Pst fragment from pLG 300 bearing the large portion of the *lacZ* gene and having a filled in Bam end.

*E. coli* were again transformed and selected for growth on ampicillin.

Two plasmids, pLG 104 and pLG 117, were then derived from the selected pLG 56 plasmids. Plasmid pLG 104 bore the *lac* promoter adjacent the ATG at the beginning of the pre-inteferon leader sequence, the pLG 117 bore the *lac* promoter adjacent the ATG at the beginning of the mature IF. The plasmids directed the synthesis of hybrid proteins having, respectively, an amino-terminal pre-IF fragment fused to a carboxy-terminal β-galactosidase fragment, and an amino-terminal mature IF fragment fused to a carboxy-terminal β-galactosidase fragment.

The two plasmids were derived as follows. First, pLG 56 was cut in front of the ATG of the pre-IF gene region (pLG 104) and reserted with Bal 31 exonuclease. The plasmids were then cut with Bam to remove the shortened promoter fragment and religated in the presence of an excess of a Bam—PvuII promoter-bearing fragment from pGL 101 B.

*E. coli* were transformed with the plasmids and selected for growth on ampicillin and for production of a marked color change on indicator agar which changes color to a degree proportional to β-galactosidase level. The clones having the promoter optimally positioned were those which produced the greatest amount of β-galactosidase. One high production clone bore a plasmid having the *lac* promoter positioned such that there were seven base pairs between the Shine-Dalgarno sequence and the ATG of the pre-interferon leader sequence (pLG 104) and another bore a plasmid having the *lac* promoter positioned such that there were seven base pairs between the Shine-Dalgarno sequence and the ATG of the mature IF gene (pLG 117). Generally, optimal expression will be obtained when there are about two to fourteen base pairs between the Shine-Dalgarno sequence and the ATG.

To construct plasmid 104 R, the 3700 base pair Pst—Pst region from TpIF 319 bearing the 3' end of the IF gene was ligated to the ~1050 base pair Pst—Pst region of the pLG 104 bearing the *lac* promoter adjacent the ATG at the beginning of pre-IF. Plasmid 117 R was constructed by ligating the same 3700 base pair region of TpIF to the ~990 base pair Pst—Pst region of pLG 117 bearing the *lac* promoter adjacent the ATG at the beginning of mature IF.

Plasmid 104 R thus comprised a fused gene including the *lac* promoter (any portable promoter having a Shine-Dalgarno sequence would have sufficed), seven base pairs, a translation start site, and the reconstituted gene coding for unglycosylated human fibroblast pre-interferon. Plasmid 117 R had the same structure except that it included the reconstituted gene for mature interferon rather than for pre-inteferon.

*E. coli* microorganisms containing the above plasmids have been deposited with the American Type Culture Collection under the numbers: *E. coli* containing plasmid 104R — ATCC No. 31902; *E. coli* containing plasmid 117 R — ATCC No. 31903.

Both plasmids produced active, unglycosylated fibroblast interferon having the same amino acid seqeunce as naturally-occurring, glycosylated human fibroblast interferon:

met-ser-tyr-asn-leu-leu-gly-phe-leu-gln-arg-ser-ser-asn-phe-gln-cys-gln-lys-leu-leu-trp-gln-leu-asn-gly-arg-leu-glu-tyr-cys-leu-lys-asp-arg-met-asn-phe-asp-ile-pro-glu-glu-ile-lys-gln-leu-gln-gln-phe-gln-lys-glu-asp-ala-ala-leu-thr-ile-tyr-glu-met-leu-gln-asn-ile-phe-ala-ile-phe-arg-gln-asp-ser-ser-ser-thr-gly-trp-asn-gly-thr-ile-val-glu-asn-leu-leu-ala-asn-val-tyr-his-gln-ile-asn-his-leu-lys-thr-val-leu-glu-glu-lys-leu-glu-lys-glu-asp-phe-thr-arg-gly-lys-leu-met-ser-ser-leu-his-leu-lys-arg-tyr-tyr-gly-arg-ile-leu-his-tyr-leu-lys-ala-lys-glu-tyr-ser-his-cys-ala-trp-thr-ile-val-arg-val-glu-ile-leu-arg-asn-phe-tyr-phe-ile-asn-arg-leu-thr-gly-tyr-leu-arg-asn.

The basic method Example 1 can be used to express, in *E. coli* strain K—12, the human leucocyte pre-interferon gene isolated and described in Mantei et al (1980) Gene *10*, 1—10.

(Bacteria are transformed at appropriate times, as described in Example 1, and selected for growth on ampicillin; these steps are omitted from the following description.)

The first step is to use complement DNA cloning, involving G—C tailing, to insert the human leucocyte pre-IF gene, diagrammatically illustrated in Fig. 5, into a suitable plasmid such as pBR 322. Next, the amino-terminal section of the pre-IF gene is removed by cutting with HinF 1, and the ends filled in using DNA polymerase (Backman et al., *supra*). Next, Hind III linkers are attached. A section of this region is then cut out using Hind III and Mb01. This section,

$$\text{Hind III} \vdash\!\!\!-\!\!\!-\!\!\!-\!\!\!\overset{\text{ATG}}{\underset{\text{pre-IF'}}{\vdash}}\!\!\!-\!\!\!-\!\!\!-\!\!\!\dashv \text{Mb01},$$

is then cloned into a pBR 322 backbone to yield the plasmid shown in Fig. 6. This plasmid is then cut with Mb01 and R1 and the ends of the resulting DNA fragment filled in using DNA polymerase (Backman et al., *supra*). The fragment is then cloned into pLG 300 (Guarente et al. Serial No. 131,152, *supra*) which has been cut with Bam and had the ends filled in, yielding the plasmid, shown in Fig. 7, having the *lacZ* gene region capable of coding for an assayable fragment of β-galactosidase.

The plasmid of Fig. 7 is then opened with Hin 3 and the ends are digested with exonuclease in order to provide a site close the ATG of the pre-IF gene in which to insert the Pst—Pvull portable promoter fragment from pGL 101. After this insertion bacteria are selected as in Example 1 for maximum β-galactosidase production. The plasmid of a high-production clone, shown diagrammatically in Fig. 8, has two to fourteen base pairs between the Shine-Dalgarno sequence and the ARG. From such a plasmid the gene for human leucocyte pre-interferon is reconstituted by cutting with Pvull and ligating the carboxy-terminal end of the pre-IF gene onto the cut end of the amino-terminal end. The resulting plasmid, shown diagrammatically in Fig. 9, thus comprises the *lac* promoter, two to fourteen base pairs, a translation start site, and the reconstituted gene coding for unglycosylated human leucocyte pre-interferon. This pre-interferon can then be processed by bacteria in the same manner as human fibroblast pre-interferon, yielding unglycosylated human leucocyte interferon having the same amino acid sequence as naturally-occurring, glycosylated human leucocyte interferon:

cys-asp-leu-pro-glu-thr-his-ser-leu-asp-asn-arg-arg-thr-leu-met-leu-leu-ala-gln-met-ser-arg-ile-ser-pro-ser-
ser-cys-leu-met-asp-arg-his-asp-phe-gly-phe-pro-gln-glu-glu-phe-asp-gly-asn-gln-phe-gln-lys-ala-pro-ala-
ile-ser-val-leu-his-glu-leu-ile-gln-gln-ile-phe-asn-leu-phe-thr-thr-lys-asp-ser-ser-ala-ala-trp-asp-glu-asp-leu-
leu-asp-lys-phe-cys-thr-glu-leu-tyr-gln-gln-leu-asn-asp-leu-glu-ala-cys-val-met-gln-glu-glu-arg-val-gly-glu-
thr-pro-leu-met-asn-ala-asp-ser-ile-leu-ala-val-lys-lys-tyr-phe-arg-arg-ile-thr-leu-tyr-leu-thr-glu-lys-lys-tyr-
ser-pro-cys-ala-try-glu-val-val-arg-ala-glu-ile-met-arg-ser-leu-ser-leu-ser-thr-asn-leu-gln-glu-arg-leu-arg-
arg-lys-glu.

## Claims

1. Plasmid pLG104R wherein 3700 base pair PstI—PstI region from TpIF319 bearing the 3' end of the interferon is ligated to the ~1050 base pair PstI—PstI region of pLG104 bearing the *lac* promoter adjacent to the ATG at the beginning of pre-interferon.

2. Plasmid pLG117R wherein 3700 base pair PstI—PastI region from TpIF319 bearing the 3' end of the interferon is ligated to the ~990 base pair PstI—PstI region of pLG117 bearing the *lac* promoter adjacent to the ATG at the beginning of mature interferon.

## Patentansprüche

1. Plasmid pLG104R, in dem der 3700 Basenpaare lange PstI—PstI-Bereich aus TpIF319, der das 3'-Ende des Interferons trägt, mit dem ~1050 Basenpaare langen Bereich des PstI—PstI-Bereich von pLG104, der den lac-Promoter neben dem ATG-Codon am Anfang des Interferon-Vorläufers (Pre-Interferon) trägt, verknüpft ist.

2. Plasmid pLG117R, in dem der 3700 Basenpaare lange PstI—PstI-Bereich von TpIF319, der das 3'-Ende von Interferon trägt, mit dem ~990 Basenpaare langen PstI—PstI-Bereich von pLG117, der den lac-Promotor neben dem ATG-Codon am Anfang von fertigem Interferon trägt, verknüpft ist.

## Revendications

1. Plasmide pLG104R dasn lequel la région PstI-PstI comportant 3700 paires de bases, provenant de TpIF319, portant l'extrémité 3' de l'interféron, est soudée à la région PstI—PstI de pLG104, comportant environ 1050 paires de bases, portant le promoteur *lac* adjacent à l'ATG de la partie initiale du pré-interféron.

2. Plasmide pIG117R dans lequel la région PstI—PstI comportant 3700 paires de bases, provenant de TpIF319, portant l'extrémité 3' de l'interféron, est soudée à la région PstI—PstI de pLG117, comportant environ 990 paires de bases, portant le promoteur *lac* adjacent à l'ATG de la partie initiale de l'interféron mature.

FIG 1

FIG 2

FIG 3

FIG 4

# FIG 5

# FIG 6

# FIG 7

FIG 8

FIG 9